# EUROPEAN PATENT SPECIFICATION

(11) **EP 0 461 085 B1**
(45) Date of publication and mention of the grant of the patent: **05.01.1994**
(21) Application number: 91830236.5
(22) Date of filing: 03.06.1991
(51) Int. Cl.: A61K 9/70, A61K 47/10, A61K 47/32

(54) **Pharmaceutical and/or cosmetic preparation**
Pharmazeutische und/oder kosmetische Zubereitung
Préparation pharmaceutique et/ou cosmétique

(30) Priority: 04.06.1990 IT 4802690
(43) Date of publication of application: 11.12.1991
(73) Proprietor: GEYMONAT S.p.A., I-03012 Anagni FR (IT)
(72) Inventor: Salvia, Giuseppe, I-95123 Catania CT (IT); Heusser, Jean, CH-8134 Adliswil (CH); Conti, Ettore, I-00040 Rocca di Papa RM (IT)
(74) Representative: Di Cerbo, Mario

(56) References cited:
- EP-A- 0 148 170
- US-A- 4 938 951
- DATABASE: WPIL, accession no. 81-70856D [39], Derwent Publications Ltd, London, GB; & JP-A-56 100 882 (NITTO ELECTRIC IND. K.K.) 13-08-1981

## Description

The present invention refers to a pharmaceutical and/or cosmetic preparation.

In order to treat the surface of human or animal skin topically, pharmaceutically and/or cosmetically, liniments, plasters, emulsions, dispersions, lotions are mainly used.

An object of the present invention is to provide a new galenic form, i.e. a film for the proportioned release of vegetable substances with pharmaceutical and/or cosmetic activity. This new form, in comparison with conventional galenic forms, should make possible a better, more uniform and higher adsorption of the active substances through the skin. At the same time, stability of the active compounds in the film should be increased.

These objects are satisfactorily achieved with the preparation according to the present invention.

The pharmaceutical and/or cosmetic preparation as per the invention is characterized by the fact that it contains, homogeneously distributed, at least one water soluble polyvinylic alcohol, at least one component A, selected from the group consisting of surfactants, non-ionic surface active products and dispersants, and moreover at least one vegetable substance having pharmaceutical and/or cosmetic activity, and optionally one or more auxiliary substance, and characterized by the fact that it is in the form of a film with a layer thickness of from 0.05 to 0.5 mm, in particular from 0.06 to 0.2 mm, preferably 0.07 mm to 0.15 mm.

The vegetable substance can contain the flavonoids of Anthyllis dillenii.

The component A can be selected from monovalent alcohols, for example a nonoxynol, such as nonoxynol-9, nonoxynol-10, and from polyvalent alcohols, for example propylene glycol, glycerin and combinations thereof, such as in particular a mixture of nonoxynol and glycerin.

The preparation according to the invention can contain from 50 to 70% by weight, in particular from 55 to 65% by weight, of a polyvinylic alcohol, from 15 to 35% by weight, in particular from 20 to 30% by weight, of nonoxynol, from 3 to 8% by weight, in particular 5% by weight of glycerin and up to 15% by weight of vegetable substance (or substances), all the above components, including the optional auxiliary substances, summing up to 100% by weight.

The auxiliary substances can be selected from the group consisting of stabilizers, plasticizers, buffers, antioxidants, perfumes and dyes, and they are each time contained in active concentrations.

The surface of the film can be increased by means of e.g. embossing.

Within the film a gauze can be present. The film can also be applied onto a gauze by pressure.

A possible preparation of the film according to the invention is disclosed hereinafter.

A water soluble, polyvinylic alcohol, for instance Povan 205 of Kuraray Co. Ltd., Japan, is mixed with at least one component A, for example a mixture of at least one nonoxynol, for example nonoxynols of formula

C₉H₁₉-C₆H₄ ( OCH₂CH₂ )ₙ OH

wherein n is an integer, preferably 9 or 10, and glycerin, and with water. The mixture is then heated, for instance at a temperature of about 90°C. The cold solution is preferably filtered, for instance at a temperature of about 50° C. If the vegetable substances and the optional auxiliary substances are soluble in water, they are dissolved in water and are added to the mixture preferably before filtration. If the vegetable substances and the optional auxiliary substances are not soluble in water, they are added to the mixture after the above filtration, preferably under agitation, for instance in the form of a suspension or an emulsion, aqueous suspensions and aqueous emulsions being preferred. Water-in-oil microemulsions and oil-in-water microemulsions can be used also. It is also possible for vegetable and/or auxiliary substances which are not soluble in water, to be added in solid form, preferably under agitation, to the filtration mixture, in particular when the suspension is homogenized. The addition to the mixture of vegetable and/or auxiliary substances dissolved in at least one organic solvent is also possible. If this kind of addition takes place, no separation of phases between organic solvent and water should occur. Said filtration is carried out in order to remove impurities, if any, such as for instance water insoluble polymers of polyvinylic alcohol, dust and other foreign particles. In the case of absolutely pure and unitary products, the filtration could be avoided. The film-generating solution should be homogeneous. In the case of air bubbles being present, they can be removed by leaving the solution to rest or by treating under vacuum or by performing a gentle agitation.

This film-generating solution can be treated in many manners, in the conventional way, in order to obtain a film.

According to a first method, the solution is batch poured into a tank which has the dimensions desired. Then, the water and organic solvents, if any, are removed by drying. The drying can be carried out, for instance, by means of hot air, coming from a suitable hair-dryer, or coming from a suitable thermic lamp, for example an infra-red ray lamp. The temperature used should be such that no component should be chemically transformed, in particular decomposed as a result of heating. If desired, a gauze, preferably a sterilized gauze, can be put onto the plate before pouring the film-generating solution thereon. After drying, a gauze-reinforced film is obtained. The film can now be subjected to an embossing, thus increasing its surface and "hand". The film is finally cut and packed, for instance into bags.

According to a second method, the film-generating solution undergoes a continuous treatment in a device for producing films. The film-generating solution is poured onto a continuous tape through a slot nozzle. The film support is usually a strip of chrome steel polished to obtain high brightness. When pouring, the viscosity of the film-generating solution should be such as to flow still, but not to stop.

The viscosity to be adopted each time depends among other things on the type of machine. The continuous tape coated with film-generating solution enters a drying tunnel where water and organic solvent, if any, are removed. The temperature in the drying tunnel should be such as to prevent any chemical transformation, in particular heat decomposition, of all the components. At the end of the drying tunnel, the film still contains only a small percent by weight of water, for instance about 5% by weight of water. The desired film thickness can be controlled and regulated by using a computer. If, when starting, the film is too thin, then the slot nozzle is automatically opened. On the contrary, if the film is too thick, the slot nozzle is closed. If desired, into the dried or slightly moistened film a gauze can be introduced under pressure, for instance by using a device in which two cylinders rotate in an opposite direction to each other. As regards embossing, cutting and type of packaging, reference is made to the relevant embodiments.

The vegetable substance with pharmaceutical and/or cosmetic activity, is preferably a liposomal extract and contains in particular the flavonoids of Anthyllis dillenii, for example "liposomi" from the company Geymonat Spa, Anagni (Frosinone), Italy, also known as Sal 29^{(R)} which act against cellulitis and, moreover, have an antiedematose effect and a fibrosclerotic effect. As regards the above action, reference can be made to the following literature:
S.B. Curri, Minerva Mesoterapeutica, vol.2 - No.1, pages 15 to 30, 1987.

The preparation according to the invention, cut to the desired dimensions, for example 10x15 cm, can be applied onto the human or animal skin. The pharmaceutical and/or cosmetic effect to be obtained depends on the combination of the effective substances employed. As a result of body heat and/or of transpiration, the active substance or substances contained in the film, in comparison with an ointment, exhibit a better and more homogeneous action.

The following example is aimed at illustrating the present invention.

### Example

A mixture of 6.81 Kg of polyvinylic alcohol Poval 205 from the firm Kuraray Co., Ltd., Japan, 560 g of glycerin and 1.582 Kg of nonoxynol-9, in 14.7 Kg of water was heated under agitation, slowly, at a temperature of 90°C. The slightly turbid solution was cooled and filtered at a temperature of 50°C. To the limpid filtrate a suspension of 500 g of Liposomi Sal 29^{(R)} in 2 Kg of water was added under agitation at a temperature of 35°C. This mixture was left overnight at a temperature of 35°C, after which no air bubbles were any longer present in the mixture. With film-generating solution, using a device for pouring the above film-generating solution, a homogenous film having a thickness of 0.06 - 0.07 mm was prepared. The film-generating solution was poured at a temperature of 35°C.

When compared with a conventional ointment, also containing Liposomi Sal 29^{(R)}, the adsorption obtained by using the film according to the present invention was better in the measure of 40%.

## Claims

1. A pharmaceutical and/or cosmetic preparation, characterized by the fact that it contains, homogeneously distributed, at least one water soluble polyvinylic alcohol, at least one component A, selected from the group consisting of surfactants, non-ionic surface active products and dispersants, and moreover at least one vegetable substance with pharmaceutical and/or cosmetic activity and optionally at least one auxiliary substance, and characterized by the fact that it is in the form of a film with a layer thickness of from 0.05 to 0.5 mm.

2. The preparation according to claim 1, in the form of a film with a layer thickness of from 0.06 to 0.2 mm.

3. The preparation according to claim 2, in the form of a film with a layer thickness of from 0.07 to 0.15 mm.

4. The preparation according to any of claims 1 to 3, in which the vegetable substance contains the flavonoids of Anthyllis dillenii.

5. The preparation according to any of claims 1 to 4, in which the component A is selected from the group comprising monovalent alcohols, polyvalent alcohols and combination thereof.

6. The preparation according to claim 5, in which the monovalent alcohol is a nonoxynol.

7. The preparation according to claim 6, in which the nonoxynol is nonoxynol-9.

8. The preparation according to claim 6, in which the nonoxynol is nonoxynol-10.

9. The preparation according to any of claims 5 to 8, in which the polyvalent alcohol is selected from the group comprising propylene glycol and glycerin.

10. The preparation according to claims 6 to 9, in which the component A is a mixture of nonoxynol and glycerin.

11. The preparation according to any of claims 1 to 10, which contains from 50 to 70% by weight, in particular from 55 to 65% by weight, of a polyvinylic alcohol, from 15 to 35% by weight, in particular from 20 to 30% by weight, of nonoxynol, from 3 to 8% by weight, in particular 5% by weight of glycerin and up to 15% by weight of at least one vegetable substance, all the above components, including the optional auxiliary substances, summing up to 100% by weight.

12. The preparation according to any of claims 1 to 11, in which the auxiliary substance is selected from the group consisting of stabilizers, plasticizers, buffers, antioxidants, perfumes and dyes, the above substance being each time contained in active concentrations.

13. The preparation according to any of claims 1 to 12, in which the surface of the film is increased by means of embossing.

14. A product, characterized by the fact of comprising the preparation according to any of claims 1 to 13 with a gauze within the film.

15. A product, characterized by the fact of comprising the preparation according to any of claims 1 to 13 applied onto a gauze by pressure.

## Patentansprüche

1. Pharmazeutische und/oder kosmetische Zubereitung, **da****durch gekennzeichnet,** daß sie homogen verteilt mindestens einen wasserlöslichen Polyvinylalkohol, mindestens eine Komponente A, ausgewählt aus der Gruppe bestehend aus oberflächenaktiven Mitteln, nichtionischen oberflächenaktiven Produkten und Dispergiermitteln, und darüber hinaus mindestens eine pflanzliche Substanz mit pharmazeutischer und/oder kosmetischer Wirkung und wahlweise mindestens eine Hilfssubstanz enthält, und **dadurch gekennzeichnet**, daß sie in Form eines Films mit einer Schichtdicke von 0,05 bis 0,5 mm vorliegt.

2. Zubereitung nach Anspruch 1, in Form eines Films mit einer Schichtdicke von 0,06 bis 0,2 mm.

3. Zubereitung nach Anspruch 2, in Form eines Films mit einer Schichtdicke von 0,07 bis 0,15 mm.

4. Zubereitung nach einem der Ansprüche 1 bis 3, in welcher die pflanzliche Substanz die Flavonoide von Anthyllis dillenii enthält.

5. Zubereitung nach einem der Ansprüche 1 bis 4, in welcher die Komponente A ausgewählt ist aus der Gruppe umfassend einwertige Alkohole, mehrwertige Alkohole und Kombinationen davon.

6. Zubereitung nach Anspruch 5, in welcher der einwertige Alkohol ein Nonoxynol ist.

7. Zubereitung nach Anspruch 6, in welcher das Nonoxynol Nonoxynol-9 ist.

8. Zubereitung nach Anspruch 6, in welcher das Nonoxynol Nonoxynol-10 ist.

9. Zubereitung nach einem der Ansprüche 5 bis 8, in welcher der mehrwertige Alkohol ausgewählt ist aus der Gruppe umfassend Propylenglycol und Glycerin.

10. Zubereitung nach den Ansprüchen 6 bis 9, in welcher die Komponente A ein Gemisch aus Nonoxynol und Glycerin ist.

11. Zubereitung nach einem der Ansprüche 1 bis 10, welche 50 bis 70 Gew.-%, insbesondere 55 bis 65 Gew.-% von einem Polyvinylalkohol, 15 bis 35 Gew.-%, insbesondere 20 bis 30 Gew.-% Nonoxynol, 3 bis 8 Gew.-%, insbesondere 5 Gew.-% Glycerin und bis zu 15 Gew.-% von mindestens einer pflanzlichen Substanz enthält, wobei alle die oben genannten Komponenten, einschließlich der wahlweisen Hilfssubstanzen zusammen 100 Gew.-% ergeben.

12. Zubereitung nach einem der Ansprüche 1 bis 11, in welchem die Hilfssubstanz ausgewählt ist aus der Gruppe bestehend aus Stabilisatoren, Weichmachern, Puffern, Antioxidationsmitteln, Duftstoffen und Farbstoffen, wobei die oben genannte Substanz jeweils in wirksamen Konzentrationen enthalten ist.

13. Zubereitung nach einem der Ansprüche 1 bis 12, in welcher die Oberfläche des Films durch Prägen vergrößert ist.

14. Produkt, **dadurch gekennzeichnet,** daß es die Zubereitung nach einem der Ansprüche 1 bis 13 mit einer Gaze in dem Film umfaßt.

15. Produkt, **dadurch gekennzeichnet,** daß es die Zubereitung nach einem der Ansprüche 1 bis 13 umfaßt, welche durch Druck auf eine Gaze aufgetragen ist.

## Revendications

1. Préparation pharmaceutique et/ou cosmétique caractérisée par le fait qu'elle contient, répartis de façon homogène au moins un alcool polyvinylique hydrosoluble, au moins un composant A choisi parmi le groupe constitué par des agents tensioactifs, des produits tensionactifs non ioniques et des agents de dispersion, et de plus au moins une substance végétale ayant une activité pharmaceutique et/ou cosmétique et facultativement au moins une substance auxiliaire, et caractérisée par le fait qu'elle se présente sous la forme d'une pellicule ayant une épaisseur de couche de 0,05 à 0,5 mm.

2. Préparation selon la revendication 1, sous forme d'une pellicule ayant une épaisseur de couche allant de 0,06 à 0,2 mm.

3. Préparation selon la revendication 2, sous forme d'une pellicule d'une épaisseur de couche allant de 0,07 à 0,15 mm.

4. Préparation selon l'une quelconque des revendications 1 à 3, dans laquelle la substance végétale contient les flavonoïdes de l'Anthyllis dillenii.

5. Préparation selon l'une quelconque des revendications 1 à 4, dans laquelle le composant A est choisi parmi le groupe comprenant des alcools monovalents, les alcools polyvalents et une combinaison de ceux-ci.

6. Préparation selon la revendication 5, dans laquelle l'alcool monovalent est un nonoxynol.

7. Préparation selon la revendication 6, dans laquelle le nonoxinol est le nonoxynol-9.

8. Préparation selon la revendication 6, dans laquelle le nonoxinol est le nonoxynol-10.

9. Préparation selon l'une quelconque des revendications 5 à 8, dans laquelle l'alcool polyvalent est choisi parmi le groupe comprenant le propylène-glycol et la glycérine.

10. Préparation selon les revendications 6 à 9, dans laquelle le composant A est un mélange de nonoxynol et de glycérine.

11. Préparation selon l'une quelconque des revendications 1 à 10, qui contient de 50 à 70 % en poids, en particulier de 55 à 65 % en poids d'un alcool polyvinylique, de 15 à 35 % en poids, en particulier de 20 à 30 % en poids de nonoxynol, de 3 à 8 % en poids, en particulier de 5 % en poids de glycérine et jusqu'à 15 % en poids d'une substance végétale, tous les composants ci-dessus, y compris les substances auxiliaires facultatives, atteignant 100 % en poids.

12. Préparation selon l'une quelconque des revendications 1 à 11, dans laquelle la substance auxiliaire est choisie parmi le groupe constitué par les stabilisants, les plastifiants, les tampons, les antioxydants, les parfums et les colorants, la substance ci-dessus étant chaque fois contenue dans des concentrations actives.

13. Préparation selon l'une quelconque des revendications 1 à 12, dans laquelle on accroît la surface de la pellicule par bosselage.

14. Produit caractérisé par le fait qu'il comprend la préparation selon l'une quelconque des revendications 1 à 13 avec de la gaze à l'intérieur de la pellicule.

15. Produit caractérisé par le fait qu'il comprend la préparation selon l'une des revendications 1 à 13 appliquée sur la gaze par pression.
